# EUROPÄISCHE PATENTSCHRIFT

(11) **EP 1 413 517 B1**
(45) Veröffentlichungstag und Bekanntmachung des Hinweises auf die Patenterteilung: **29.08.2007**
(21) Anmeldenummer: 04001697.4
(22) Anmeldetag: 20.04.2000
(51) Int. Cl.: B65B 3/00, A61M 5/30

(54) **Verfahren zum Befüllen von nadelfreien Injektoren**
Method for filling needle-free injectors
Procédé pour remplir des injecteurs sans aiguille

(30) Priorität: 24.04.1999 DE 19918721
(43) Veröffentlichungstag der Anmeldung: 28.04.2004
(62) Teilanmeldung aus: 00927019.0
(73) Patentinhaber: Inova Pharma Systems GmbH, 74523 Schwäbisch Hall (DE)
(72) Erfinder: Bernd Laukenmann, 74564 Crailsheim (DE); Michael Nikolaus Müller, 74635 Westernach (DE); Jürgen Rothbauer, 74545 Michelfeld (DE); Dr.Georg Alfred Kallmwyer, 68259 Mannheim (DE); Theo Arnitz, 68753 Waghäusel (DE)
(74) Vertreter: Patentanwälte Ruff, Wilhelm, Beier, Dauster & Partner

(56) Entgegenhaltungen:
- WO-A-97/22375
- WO-A-97/36785
- DE-A- 4 320 098
- DE-B- 1 215 541
- FR-A- 889 757
- US-A- 4 338 980

## Beschreibung

Nadelfreie Injektoren, capsules oder dergleichen werden in der Regel von der Seite aus befüllt, von der aus der Kolbenstopfen eingesetzt wird. Es gibt aber auch Fälle, in denen die Befüllung von der Seite der Austrittsöffnung her erfolgen soll, also durch die Austrittsöffnung hindurch. Im folgenden wird für diese Art von Behältnissen nur der Ausdruck Injektoren verwendet, der auch die andere Behältnisse umfassen soll

Es ist bereits ein Verfahren zum Befüllen von nadelfreien Injektoren bekannt (WO 97/36785), bei dem in den Injektor ein Kolbenstopfen eingesetzt und von der Seite der Austrittsöffnung her mit Hilfe einer Füllnadel die Flüssigkeit injiziert wird. Durch das Eindringen der Flüssigkeit wird der Kolbenstopfen verschoben.

Bei einem ähnlichen Verfahren (WO 97/22375) wird ebenfalls der Kolbenstopfen soweit in den Injektor eingeschoben, bis er zur Anlage im Bereich der Ausgabeöffnung gelangt. Nur der noch verbleibende Bereich außerhalb des Injektorraums wird evakuiert, bevor die Flüssigkeit injiziert wird.

Der Erfindung liegt die Aufgabe zugrunde, ein Verfahren zum exakten Befüllen auch unterschiedlicher Gebinde zu schaffen. Die Gebinde sollen randvoll und zumindest annähernd blasenfrei befüllt werden können.

Zur Lösung dieser Aufgabe schlägt die Erfindung ein Verfahren mit den Merkmalen des Anspruchs 1 und ein Verfahren mit den Merkmalen des Anspruchs 4 vor. Weiterbildungen der Erfindung sind Gegenstand der abhängigen Ansprüche, deren Wortlaut ebenso wie der Wortlaut der Zusammenfassung durch Bezugnahme zum Inhalt der Beschreibung gemacht wird. Unabhängig von Toleranzen des Injektors, des Kolbenstopfens, des Anschlags oder dergleichen wird der Innenraum des Injektors also immer vollständig gefüllt, teilweise sogar ohne dass eine Dosiereinrichtung die Menge der einzubringenden Flüssigkeit messen muss. Das Verfahren ist bei unterschiedlich großen Injektoren auch ohne Umrüstung anwendbar.

Erfindungsgemäß kann die Befüllung des Injektorgehäuses mittels der sogenannten Saugfüllung bei Atmosphärendruck oder geringem Überdruck der Flüssigkeit erfolgen.

Erfindungsgemäß ist vorgesehen, dass der Kolbenstopfen vor dem Beginn des Evakuierens auf eine dem gewünschten Füllvolumen entsprechende Position gebracht wird. In dieser Position wird der Kolbenstopfen während des Evakuierens und während des Befüllens festgehalten. Wenn ein stramm sitzender Kolben verwendet wird, sind keine weiteren Maßnahmen erforderlich, um den Kolben in dieser Position zu halten. Die Position selbst kann durch einen Anschlag bestimmt werden.

Erfindungsgemäß kann vorgesehen sein, dass zur Ermittlung des Zustandes, dass der Innenraum des Injektors vollständig gefüllt ist, das Verschwinden des Vakuums benutzt wird.

Nach Befüllen des Injektors kann vorgesehen sein, die Austrittsöffnung durch einen Stopfen zu verschließen.

Bei der Dimensionierung des Systems ist darauf zu achten, dass pharmazeutische Gesichtspunkte berücksichtigt werden. Sterilfilter sind an allen produktberührenden Zu- und Abgängen des Systems vorsehbar.

Weitere Merkmale, Einzelheiten und Vorzüge der Erfindung ergeben sich aus der folgenden Beschreibung der Erfindung sowie anhand der Zeichnung. Hierbei zeigen:
- Fig. 1: schematisch die Anordnung einer Vorrichtung zur Saugfüllung nach der Erfindung;
- Fig. 2: einen Teil der Vorrichtung nach Fig. 1, nämlich eine Vakuumglocke und eine Füllnadel;
- Fig. 3: eine Halterung für einen zu befüllenden Injektor;
- Fig. 4: bis 9 die verschiedenen Stadien der Vorrichtung während des Befüllens eines Injektors.

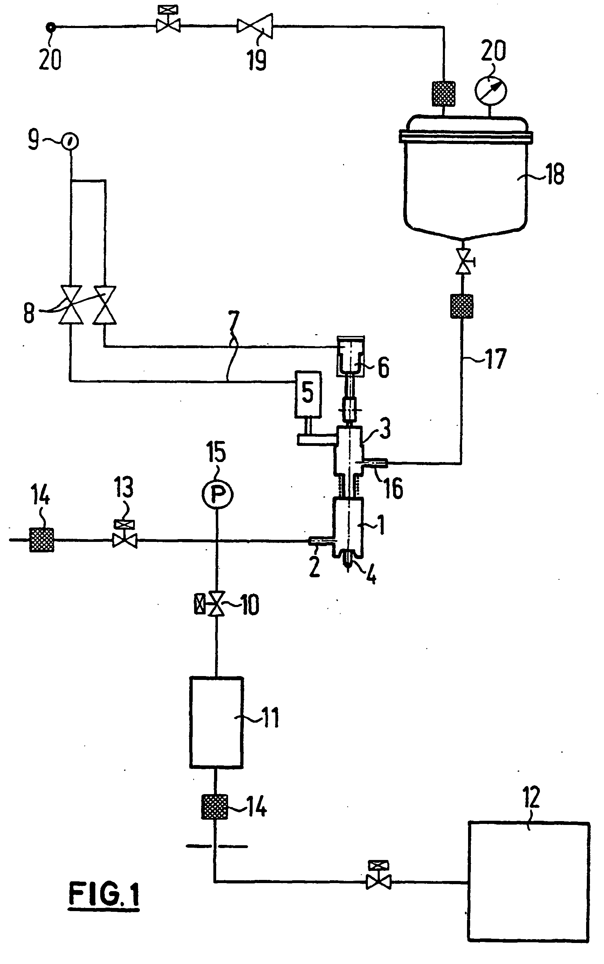

Figur 1 zeigt eine Übersicht über eine Anlage zur Saugfüllung, in der eine Vorrichtung nach der Erfindung angeordnet ist. Die eigentliche Vorrichtung ist in der Mitte der Figur 1 dargestellt. Sie enthält eine Vakuumglocke 1, die mit einem Anschluss 2 für Unterdruck versehen ist. Die Vorrichtung enthält weiterhin eine Befülleinrichtung 3, die eine Füllnadel 4 enthält. Die Füllnadel 4 ragt an der Unterseite der Vakuumglocke 1 aus dieser heraus. Der zu befüllende Injektor, der in der Fig. 1 nicht dargestellt ist, wird unmittelbar unterhalb der Vakuumglocke 1 und der Füllnadel 4 angeordnet. Die Füllnadel 4 und die Vakuumglocke 1 können zusammen durch einen ersten Antrieb 5 abgesenkt und wieder angehoben werden. Dieser Antrieb 5 kann beispielsweise druckluftgesteuert sein. Mit Hilfe eines zweiten koaxialen Antriebs 6 kann eine Verschlusseinrichtung die Füllnadel 4 öffnen und schließen. Zur Ansteuerung dieser beiden Antriebe sind Druckleitungen 7 vorhanden, die über Steuerventile 8 mit einer Druckluftquelle 9 in Verbindung stehen.

Der Anschluss 2 für Unterdruck steht über ein Ventil 10 mit einem Vakuumtank 11 in Verbindung, in dem das Vakuum durch eine Vakuumpumpe 12 aufrechterhalten wird. Der Anschluss 2 kann durch ein weiteres Ventil 13 belüftet werden. In allen Leitungen sind Sterilfilter 14 vorhan den, da es sich bei der abzufüllenden Flüssigkeit in der Regel um medizinische Flüssigkeiten handelt. Der in der zu dem Anschluss 2 führenden Leitung herrschende Druck kann mit Hilfe einer Messeinrichtung 15 gemessen werden.

Die Füllnadel 4 weist einen Anschluss 16 für die einzufüllende Flüssigkeit auf. Dieser Anschluss 16 steht über eine Leitung 17 mit einem Tank 18 in Verbindung, in dem die zu befüllende Flüssigkeit untergebracht ist. Zur Herstellung eines Druckes, der die Flüssigkeit befördert, kann über ein Regelventil 19 eine Verbindung zu einer Druckluftquelle 20 hergestellt werden. Der in dem Tank 18 herrschende Druck kann ebenfalls mit Hilfe eines Messgeräts 20 gemessen werden.

Die verschiedenen Ventile können über eine entsprechende nicht dargestellte Steuerung geöffnet und geschlossen werden.

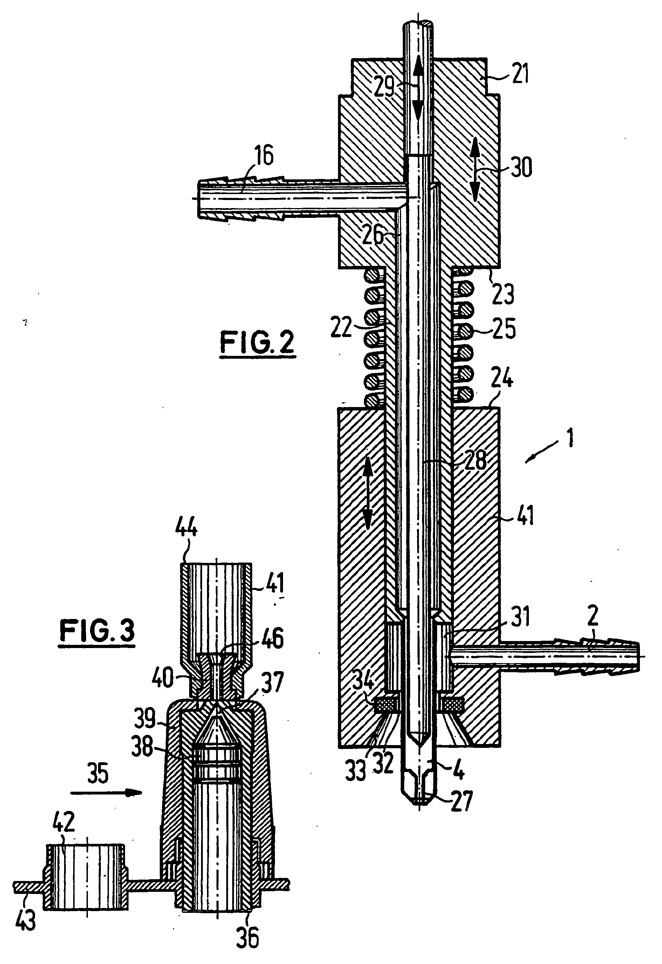

Figur 2 zeigt in größerem Maßstab Einzelheiten der Vakuumglocke 1 und der Füllnadel 4. An einem Bauteil 21, in das der Anschluss 16 für die einzufüllende Flüssigkeit mündet, ist ein hohler zylindrischer Ansatz 22 angebracht, an dessen zylindrischer Außenseite die Vakuumglocke 1 verschiebbar ge-führt ist. Zwischen einer Schulter 23 des Bauteils 21 und einer dieser gegenüberliegenden Stirnfläche 24 der Vakuumglocke 1 erstreckt sich eine Feder 25. Durch den hohlzylindrischen Ansatz 22 erstreckt sich ein Rohr 26, das in dem Bereich seines freien Endes in die Füllnadel 4 übergeht. Die Füllnadel 4 weist im Bereich ihres freien Endes, unten in Figur 2, eine Öffnung 27 mit einem deutlich kleineren Durchmesser als ihr Außendurchmesser auf.

Das Rohr 26 steht mit dem Anschluss 16 in Verbindung.

Durch das Rohr 26 und die Füllnadel 4 erstreckt sich eine Verschlussstange 28, die durch eine seitliche Öffnung im Bereich des oberen Endes des Rohres 26 durch die Wand der Zuleitung für die Flüssigkeit hindurchgeht. Dort erfolgt eine nicht näher dargestellte Abdichtung. Die Stange ist mit dem bereits erwähnten und in Figur 1 dargestellten Antrieb 6 verbunden und kann in Richtung des Doppelpfeils 29 bewegt werden.

Der Antrieb 5 kann das Bauteil 21 und damit die Füllnadel 4 in Richtung des Doppelpfeils 30 bewegen. Bei der Abwärtsbewegung des Bauteils 21 und damit der Füllnadel 4 wird gleichzeitig auch die Vakuumglocke 1 mit bewegt. Erst dann, wenn die Vakuumglocke 1 gegen einen Körper anschlägt, bleibt sie stehen, während die Füllnadel 4 weiter bewegt werden kann.

Der Anschluss 2 für Unterdruck mündet in einem die Füllnadel umgebenden Hohlraum 31, von dem aus durch einem schmalen ringförmigen Spalt 32 Luft aus der Umgebung der Füllnadel von außerhalb der Vakuumglocke angesaugt werden kann. Die Vakuumglocke 1 weist eine diesen Spalt 32 umgebende Kegelstumpffläche 33 auf, an deren dem Spalten 32 zugewandten Endes eine Dichtung 34 angeordnet ist.

Die in Figur 2 dargestellte Einrichtung arbeitet mit dem in Figur 3 dargestellten Injektor 35 zusammen. Der Injektor enthält einen Glaskörper 36, der zur Aufnahme der Flüssigkeit bestimmt ist und einen zylindrischen Innenraum 37 aufweist. In dem Innenraum ist ein Kolbenstopfen 38 angeordnet, der drei umlaufende Dichtungen aufweist und dadurch in dem Innenraum gehalten ist. An der in Figur 3 oberen Seite geht der zylindrische Innenraum 37 in einen kegelförmigen Raum über, der über eine relativ kleine Austrittsöffnung nach außen führt.

Der Glaskörper 36 mit dem Kolbenstopfen 38 ist in einer äußeren Kunststoffhülse 39 eingesetzt, die eine Halterung für eine zu der Austrittsöffnung führende Dichtungshülse 40 bildet. Auf der dem Glaskolben 36 abgewandten Seite geht die Kunststoffhülse 39 in eine weitere Hülse 41 mit einem etwas kleineren Außendurchmesser über.

Der Injektor 35 steckt in einer Hülse 42 eines Aufnahmenestes 43, das zum Transport und zur Halterung der Injektoren 35 dient. Das Aufnahmenest ist plattenförmig ausgebildet und dient zur Aufnahme mehrerer Injektoren 35.

Die Stirnkante 44 der dem Glaskörper 36 abgewandten Hülse 41 ist so bemessen, dass sie zur Anlage an der Unterseite der Dichtung 34 der Vakuumglocke 1 bestimmt und geeignet ist. Zum Befüllen wird die Vakuumglocke 1 auf die Hülse 41 abgesenkt, bis diese Stirnkante 44 an der Dichtung 34 anliegt. Durch die Dichtungshülse 40 erfolgt das Evakuieren des Innenraumes des Glaskörper 36 zwischen dessen Austrittsöffnung und dem Kolbenstopfen 38. Dann kann die Füllnadel 4 bis in Kontakt mit der Dichtungshülse 40 bewegt werden. Durch die Dichtungshülse 40 erfolgt ebenfalls das Befüllen des Glaskörpers 36.

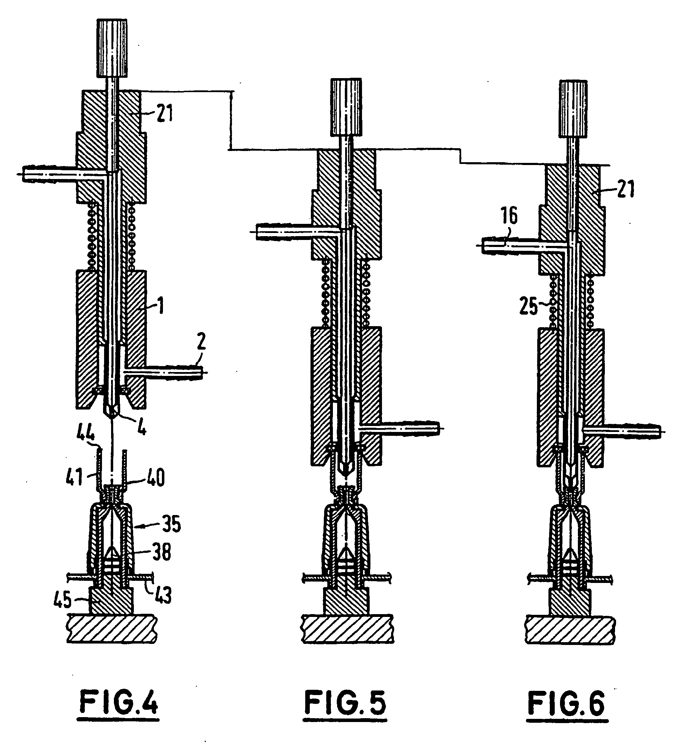

Der Verfahrensablauf wird nun anhand der Figur 4 bis 9 dargestellt und beschrieben, die die verschiedenen Stadien des von der Erfindung vorgeschlagenen Verfahrens darstellen.

Figur 4 zeigt die Ausgangsposition eines möglichen Verfahrens. Der Injektor 35 ist in dem Aufnahmenest 43 gehaltert. Er ist so auf einen Anschlag 45 aufgesetzt, dass der Kolbenstopfen 38 eine bestimmte Position einnimmt, die der gewünschten Sollposition des Kolbenstopfens 38 entspricht. Die Austrittsöffnung und die Dichtungshülse 40 sind axial gegenüber der Füllnadel 4 ausgerichtet. Durch Betätigen des Antriebs 5 wird das Bauteil 21 mit der Füllnadel 4 und der Vakuumglocke 1 abgesenkt. Diese Bewegung wird fortgesetzt, bis die Dichtung 34 auf der Stirnkante 44 so aufliegt, dass die Feder vorgespannt ist. Damit können Höhenunterschiede zwischen den Kapsulen ausgeglichen werden. Dies ist in Figur 5 dargestellt. Nun wird der Anschluss 2 unter Unterdruck gesetzt, so dass die Luft aus dem Innenraum 37 oberhalb des Kolbenstopfens 38 durch den Ringspalt 32 abgesaugt wird. Der abgedichtete Innenraum der Hülse 41 und das Innere des Glaskörpers 36 werden evakuiert. Sobald die Evakuierung abgeschlossen ist, also das Vakuum einen ausreichend hohen Wert erreicht, wird das Bauteil 21 weiter abgesenkt, wodurch die Feder 25 gespannt und die Füllnadel 4 in Kontakt mit der äußeren Öffnung 46 der Dichtungshülse 40 gebracht wird. Dieser Zustand ist in Figur 6 dargestellt. Nun wird durch Betätigen des Antriebs 6 die Verschlussstange 28 angehoben, so dass die Öffnung 27 der Füllnadel 4 geöffnet wird. Auch dies ist in Figur 6 dargestellt. Die Flüssigkeit kann nun durch den Anschluss 16 und die Füllnadel 4 in den evakuierten Innenraum 37 des Glaskörpers 36 strömen. Sobald das Vakuum in diesem Innenraum beseitigt ist, ist der Innenraum befüllt.

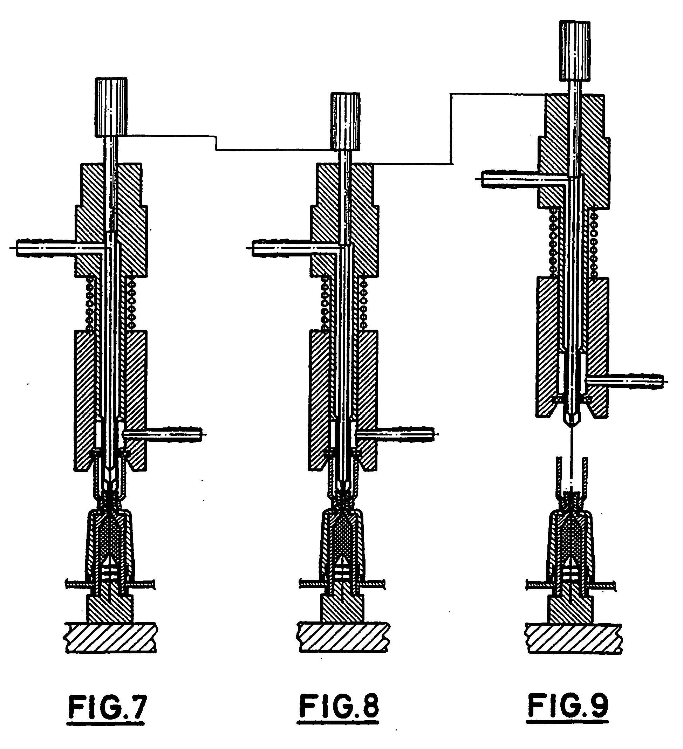

Sobald der Innenraum 37 befüllt ist, siehe Figur 7, wird ein Bypass geöffnet, um einen ggf. vorhandenen Überdruck abzubauen. Die Verschlussstange 28 wird wieder abgesenkt, bis sie die Öffnung 27 verschließt. Dies zeigt die Figur 8. Spätestens zu diesem Zeitpunkt wird der von der Hülse 41 begrenzte Raum wieder über die Vakuumglocke 41 belüftet, damit die Vakuumglocke 1 wieder von dem Injektor 35 abgehoben werden kann. Anschließend wird die Einheit aus Vakuumglocke 1 und Füllnadel 4 angehoben, so dass der gefüllte Injektor 35 weitertransportiert werden kann.

Bei dem unter Bezugnahme auf die Figuren 4 bis 9 beschriebenen Verfahren war der Kolbenstopfen 38 in seiner endgültigen Sollposition angeordnet worden, bevor die Evakuierung und Befüllung begann.

## Patentansprüche

1. Verfahren zum Befüllen von nadelfreien Injektoren (35), capsules oder dergleichen, bei dem
1.1 in den Injektor (35) ein Kolbenstopfen (38) eingesetzt wird,
1.2 die Flüssigkeit durch die Austrittsöffnung eingefüllt und
1.3 bei vollständig befülltem Innenraum (37) des Injektors (35) die Befüllung beendet wird,
**dadurch gekennzeichnet, dass**
1.4 der Kolbenstopfen (38) auf eine dem gewünschten Füllvolumen entsprechende Position positioniert,
1.5 danach der Innenraum (37) des Injektors (35) von der Seite der Austrittsöffnung her evakuiert wird, und
1.6 der Kolbenstopfen (38) während des Evakuierens und Befüllens festgehalten wird.

2. Verfahren nach Anspruch 1, bei dem die Befüllung mit Atmosphärendruck oder geringem Überdruck erfolgt.

3. Verfahren nach einem der vorhergehenden Ansprüche, bei dem zur Ermittlung des Füllungszustandes das Verschwinden des Vakuums benutzt wird.

4. Verfahren nach einem der vorhergehenden Ansprüche, bei dem die Austrittsöffnung des Injektors (35) nach der Befüllung verschlossen wird.

## Claims

1. A method of filling needle-free injectors (35), capsules, or the like,
1.1 with which a piston plug (38) is provided in the injector (35),
1.2 and liquid is filled in through the ejection opening,
1.3 and which ends the filling when the inner area (37) of the injector (35) is completely filled,
**characterized in that**
1.4 the piston plug (38) is positioned in the position corresponding to the desired filling volume,
1.5 afterwards the inner area (37) of the injector (35) is evacuated from the side of the ejection opening, and
1.6 the piston plug (38) is held during evacuation and filing.

2. A method according to claim 1, with which the filling is effectuated under atmospheric or small overpressure.

3. A method according to one of the preceding claims, with which the absence of vacuum is used to determine the filling status.

4. A method according to one of the preceding claims, with which the ejection opening of the injector (35) is closed after filling.

## Revendications

1. Procédé de remplissage d'injecteurs (35), capsules ou analogues sans aiguille, dans lequel
1.1 un bouchon de piston (38) est inséré dans l'injecteur (35),
1.2 le liquide est introduit par l'orifice de sortie et
1.3 lorsque l'espace intérieur (37) de l'injecteur (35) est rempli, le remplissage prend fin,
**caractérisé en ce que**
1.4 le bouchon de piston (38) est positionné sur une position correspondant au volume de remplissage souhaité,
1.5 puis, l'espace intérieur (37) de l'injecteur (35) est évacué par le côté de l'orifice de sortie et
1.6 le bouchon de piston (38) est maintenu durant l'évacuation et le remplissage.

2. Procédé selon la revendication 1, dans lequel le remplissage s'effectue à l'aide de la pression atmosphérique ou d'une légère surpression.

3. Procédé selon l'une des revendications précédentes, dans lequel la disparition du vide sert à générer l'état de remplissage.

4. Procédé selon l'une quelconque des revendications précédentes, dans lequel l'orifice de sortie de l'injecteur (35) est fermé après le remplissage.
